# EUROPEAN PATENT SPECIFICATION

(11) **EP 0 530 861 B1**
(45) Date of publication and mention of the grant of the patent: **09.09.1998**
(21) Application number: 92119054.2
(22) Date of filing: 21.04.1987
(51) Int. Cl.: A61K 31/20, A61K 31/23

(54) **Topical antimicrobial pharmaceutical compositions**
Topische antimikrobielle Arzneimittel
Compositions pharmaceutiques topiques antimicrobiennes

(30) Priority: 21.04.1986 US 854154
(43) Date of publication of application: 10.03.1993
(62) Divisional of application: 87303487.0
(73) Proprietor: Kabara, Jon Joseph, Galena Illinois 61036 (US)
(72) Inventor: Kabara, Jon Joseph, Galena Illinois 61036 (US)
(74) Representative: Sexton, Jane Helen

(56) References cited:
- EP-A- 0 068 552
- EP-A- 0 244 144
- FR-A- 2 097 036
- US-A- 4 002 775
- PATENT ABSTRACTS OF JAPAN vol. 9, no. 82 (C-275)11 April 1985
- PATENT ABSTRACTS OF JAPAN vol. 1, no. 54 (C-014)25 May 1977
- LIPIDS vol. 12, no. 9, pages 753 - 9 KABARA ET AL.
- Patent Abstracts of Japan,vol 9,no.82,(c-275);translation of JP-A 59216577.

## Description

The present invention relates generally to topical pharmaceutical compositions and, more particularly, to primarily aqueous solutions exhibiting unique antimicrobial properties.

The use of antimicrobial agents plays an important part in current medical therapy. This is particularly true in dermatology, where the most effective course of treatment for skin, mucous membranes, hair lesions, or infections frequently includes the use of a topical antimicrobial agent. The present invention includes topical compositions and uses which provide unique performance against a broad spectrum of microbes with excellent speed.

Various carboxylic acids are known to be good antimicrobial agents. However, when these acidic solutions are directly applied to an animal's skin, they may eg. interfere with wound healing or cause irritation or inflammation. Also, these solutions have poor solubility properties in water which, in turn, limits the efficacy of aqueous solutions employing these materials.

It is also well recognized that there is presently a need for a mild antimicrobial which can be safely applied to skin and mucous membranes, such as an animal's teats, ears and eyes, that will be substantially harmless to the applied area but which will eliminate, arrest, or substantially reduce the growth of eg bacteria, fungi or mold. The instant invention meets this need.

The art discloses that several different carboxylic acid are generally useful in the suppression of growths such as fungi, bacteria and molds. United States Patent No. 4,406,884 issued to Fawzi discloses a topical antimicrobial composition in the form of an aqueous gel or lotion. This composition contains C₅-C₁₂ fatty acids and has a pH no greater than about 5. United States Patent No. 4,343,798 issued to Fawzi, teaches a topical antimicrobial anti-inflammatory composition having a pH less than about 5 and containing C₅-C₁₂ fatty acids together with a corticosteroid component. United States Patent No. 4,489,097 issued to Stone, teaches the addition of anti-fungal/antibacterial materials to sterile compositions. The antifungal/antibacterial material disclosed is a C₄-C₉ carboxylate antimicrobial agent having a pH of about 6.0 or below. United States Patent No. 4,410,442 issued to Lucas, et al. teaches solutions for use with hydrophilic soft contact lenses containing C₅-C₁₂ fatty acids, especially octanoic acid. United States Patent No. 4,392,848 issued to Lucas, et al. teaches a catheter having a liquid reservoir of an antimicrobial agent flowing through the lumen of the catheter. The antimicrobial agent disclosed is a straight-chain carboxylic acid or carboxylic acid salt having a C₄-C₉ chain. United States Patent No. 4,430,381 issued to Harvey, et al. teaches a process for imparting antimicrobial properties to a material. The antimicrobial being a C₃-C₁₂ alkane, alkene or alkyne monocarboxylate. United States Patent Nos. 4,343,788 and 4,479,795, both issued to Mustacich, et al. teach medical polymers that provide diffusion for certain carboxylate antimicrobial agents. United States Patent No. 4,002,775 issued to Kabara teaches a food grade microbicidal composition having a monoester with a C₁₂ aliphatic fatty acid as its primary microbicide. United States Patent No. 1,772,975 issued to Weiland teaches the use of lactic acid, acetic acid, or combinations thereof, as antiseptics at properly adjusted pH levels. United States Patent No. 2,154,449 issued to Hoffman et al. teaches the use of aliphatic C₃-C₁₂ carboxylic acids and their salts as mold inhibitors in food compositions. United States Patent No. 2,190,714 issued to Hoffman, et al. teaches the addition of a C₃-C₁₂ carboxylic acid to inhibit growth food products other than margarine and sourdough bread. United States Patent No. 3,404,987 to Kooistra, et al. teaches an antimicrobial containing edible mineral salt and edible acid preservative substances, particularly propionic acid. United States Patent No. 2,466,663 issued to Russ, et al. teaches the use of a topical or intravenous caprylic acid solution to combat mycotic infections or growths. United States Patent No. 2,729,586 issued to Peck teaches a therapeutic composition having at least one salt of a C₃-C₁₁ monocarboxylic acid and water soluble chlorophyll.

Other materials also disclose the use of fatty acids for the suppression of eg fungi, bacteria or mold. Kabara, J., Medium-chain Fatty Acids and Esters as Antimicrobial Agents, Cosmetic and Drug Preservation, Pgs. 275-304, 1984, teaches the use of C₆-C₂₂ saturated and unsaturated fatty acids as antimicrobials. Kabara, J., Toxicological, Bactericidal and Fungicidal Properties of Fatty Acids and Some Derivatives, The Journal of the American Oil Chemists' Society, Vol. 56, No. 11, Pages 760A-767A (1979) teaches the applying of fatty acids to animal skin and eyes. Some fatty acids were found to be skin and eye irritants. Kabara, J., Inhibition of Staphlococlus Aureus In a Model Agar-Meat System By Monolaurin: A Research Note, Journal of Food Safety, Vol. 6, Pgs. 197-201 (1984), teaches the use of monolaurin as a food preservative to combat microorganisms. Kabara, J., Antimicrobial Agents Derives from Fatty Acids, JAOCS, Vol. 61, No. 2, Pgs. 397-403 (1984) teaches the use of saturated and unsaturated fatty acids as antimicrobial agents. Kabara, J., GRAS Antimicrobia Agents for Cosmetic Products, Journal of the Society of Cosmetic Chemists, Vol. 31, Pgs. 1-10 (1980), teaches the composition of monolaurin, a phenol, di-tert-butyl anisole, and a chelating agent such as ethylenediaminetetracetic acid to be useful in destroying gram positive and gram negative bacteria. Schemmel, R., Lynch, P., Krohn, K., and Kabara, J., Monolaurin as an Anticaries Agent, teaches the use of glycerol-monolaurin in inhibiting development of smooth surface caries in rats innoculated with Streptococcus mutants. Kabara, Jr., Ohkawa, M., Ikekawa, T., Katori, T., and Mishikawa, Y., Examination on Antitumor, Immunological and Plant-Growth Inhibitory Effects of Monoglycerides of Caprylic, Capric, and Lauric Acids and Related Compounds, Pharmacological Effects of Lipids, Volume II, Pgs. 263-272 (1985) teaches the use of the monoglycerides or caprylic, capric and lauric acids for regulating antitumor, immunological, and plant-growth activity. Li, C., and Kabara, J., Effects of Lauricidin on Fomes Annosus and Phellinus Weirii, AOCS Monograph No. 5, Pgs. 45-47 (1978) teaches the use of monolaurin in combating root rot fungi in coniferous forest. Kenney, D., Cosmetic Formulas Preserved With Food-Grade Chemicals, Cosmetics and Toiletries, Part 1, Vol. 97, Pgs. 71-76 (1982) and Kabara, J. and Wernette, C., Cosmetic Formulas Preserved with Food-Grade Chemicals, Cosmetics and Toiletries, Part II, Vol. 97, Pgs. 77-84 (1982) teaches the use of monoglyceride emulsifier, food-grade phenols and a chelator in the preservation of cosmetics. Kabara, J., A New Preservative System For Food, Journal of Food Safety, Volume 4, Pgs. 13-25 (1982) teaches the use of monolaurin, a food grade phenolic, and a chelator as an antimicrobial for the preservation of food. Branan, A. and Davison, P. Antimicrobials in Foods, Marcel Dekker, New York 1983, Pgs. 109-140 teaches the use of saturated, unsaturated and esters of fatty acids as antimicrobials and the use of these compounds for food preservation. Kabara, J., Fatty Acids and Derivatives as Antimicrobial Agents - A Review, AOCS Monograph No. 5, Pgs. 1-14 (1978) teaches the use of saturated, unsaturated and esters of fatty acids as antimicrobials and the use of these compounds for permeating microorganism cellular membranes for killing the microorganism.

The art also teaches many methods of ethoxylation. Nonionic Surfactants, Schick, M.J., Marcel Dekker, Inc., New York (1966) and Dillan, K., Effects of the Ethylene Oxide Distribution on Nonionic Surfactant Properties, JAOCS, Vol. 62, No. 7, Pgs. 1144-1151 (1985) teach the ethoxylation of primary alcohols to produce nonionic surfactants.

JP-A-59216577 describes a food preservative comprising (i) a C₆₋₁₂ fatty acid or its monoester of glycerol, sucrose, sorbitan and/or propylene glycol (ii) a C₁₄₋₁₈ saturated fatty acid and (iii) the monoester of (ii) with glycerol and/or propylene glycol.

The above discussion clearly reflects the ambiguous state of the art with regard to the suitability and selection of fatty acid-based materials as antimicrobials, especially in the topical or preservative mode. The art disclosed materials vary widely in their efficacy and possess an even wider variety of side effects, particularly when employed in veterinary topical materials under adverse or stressful conditions. (The term glyceryl and glycerol are used interchangeably herein when describing fatty acid esters).

The present invention relates to preservative pharmaceutical compositions and uses, especially those useful when applied topically and/or those which exhibit good shelf stability. The present invention relates to the discovery that the overall antimicrobial efficacy and pharmaceutical acceptability of certain glycerol fatty acid esters can be dramatically increased by either (1) the addition of certain ether groups, particularly ethoxy and propoxy units; (2) by use in combination with select fatty acid materials, (3) a combination of (1) and (2). Such modified glycerol fatty acid ester materials retain or exhibit an improved overall spectrum and speed of activity while producing fewer or reduced (in severity) side effects. Such a material is especially useful when compared to the unmodified material in that it produces much less irritation at the site of application.

The present invention further relates to the discovery that the spectrum and speed of activity of both modified and unmodified fatty acid esters can be significantly improved when used in a tertiary mixture or in combination with a mixture of two or more C₆-C₁₈ fatty acids, preferably C₆-C₁₂ fatty acids. All these materials are in turn employed in combination with a topical carrier. Such materials provide effective topical antimicrobial activity and are accordingly useful in the treatment and prevention of conditions which are caused, or aggravated by microbial organisms (including viruses) on skin and mucous membranes or are otherwise related to microbes.

Also, the compositions of the present invention exhibit exceptional preservative applications. In addition to preserving the final composition and stabilizing the material to increase the efficacy in cold climate or conditions, the present compositions provide outstanding preservative characteristics when added primarily as a preservative in food stuffs, cosmetic formulations and pharmaceutical compositions (topical; parenteral; intramuscular; and intravenous). The preservative applications are further discussed in EP-A-0 244 144.

For example, such compositions are useful in veterinary applications as a teat dip, an eye medication and an ear medication. A safe and effective amount of the compositions, described above, to an animal in need of such treatment on the area to be treated one or more times daily.

The glyceryl fatty acid ester for use in the compositions and uses of the present invention is preferably selected from the group consisting of polyhydric alcohols, polyglycerols, sucrose, glucose, sorbitol, propylenediol and glycerol fatty acid esters having about six to about twenty-one carbon atoms. Such preferred compounds include monocaprylin, monocaprin, monolaurin, monomyristin, monopalmitolein, α-monopalmitin, monostearin, monoolein, 1-monolinolein, 1-monolinolenin, and mixtures thereof. Still more preferred are monocaprylin, monocaprin, monolaurin, monomyristin, monopalmitolein, monolein, monoicosein and monoerucin and mixtures thereof. The highly preferred compounds include monocaprylin, monocaprin and monolaurin and mixtures thereof. Monolaurin is most highly preferred taking into account cost, availability and activity.

As discussed above, it has been further observed that a combination of a glyceryl fatty acid ester compound (either ethoxylated/propoxylated and non-ethoxylated/propoxylated) in a mixture with at least one and preferably two or more acids selected from the group consisting of C₆-C₁₈ fatty acids also demonstrates remarkable efficacy. Also, other polyols such a eg. polyglyceryl, sucrose, glucose and sorbitol sugar esters have been found to work satisfactorily when substituted for the glyceryl fatty acid ester. The useful glyceryl fatty acid esters include those selected from the groups consisting of glyceryl fatty acid esters having six to twenty-one carbon atoms and fatty acids having six to eighteen carbon atoms. The preferred glyceryl fatty acid ester compounds include monocaprylin, monocaprin, monolaurin, monomyristin, monopalmitolein, α-monopalmitin, monostearin, monolein, 1-monolinolein, 1-monolinolenin, and mixtures thereof. Still more preferred compounds include monocaprylin, monocaprin, monolaurin, monomyristin, monopalmitolein, monoolein, monoicosenoin, and monoerucin and mixtures thereof. The highly preferred compounds include monocaprylin, monocaprin, and monolaurin and mixtures thereof. The tertiary mixtures useful herein comprise a glyceryl fatty acid ester; a first fatty acid compound; and a second fatty acid compound. The preferred first and second fatty acid compounds for use in such tertiary mixtures or combinations are straight chain materials and include, without limitation, caproic, heptanoic, caprylic, pelargonic, capric, undecanoic, lauric, myristic, palmitic, heptadecanoic and stearic. The most preferred materials include carproic, heptanoic, caprylic, capric, undecanoic, and lauric. Highly preferred materials include heptanoic, caprylic and capric.

The glyceryl fatty acid esters, first fatty acid, and second fatty acid are added to a pharmaceutically-acceptable topical carrier in safe and effective amounts. They are present at a wt:wt ratio of ester: total fatty acid compounds of 1:10 to 1:5 with the glyceryl ester being present at a level of 0.5 to 5.0%; more preferably 0.1 to 2.0%; and still more preferably 0.5 to 1.0% of the final composition.

The compositions of the present invention can be prepared and applied in any suitable form, but are preferably prepared in forms including, without limitation eg. aqueous solutions, lotions, balms, gels, salves, ointments, boluses and suppositories. Accordingly, the composition and uses may additionally employ conventional compatible pharmaceutically-acceptable carrier materials useful for such applications. It is desirable that the carrier selected be capable of codissolving or suspending the materials used in the composition. Carrier materials suitable for use in the instant composition, therefore, include those well-known for use in the cosmetic and medical arts as a basis for eg. ointments, lotions, creams, salves, aerosols, suppositories or gels. A preferred carrier generally comprised of alcohols, chelating agents, surfactants, parabens and water.

The alcohols useful in the compositions and uses of the present invention may be selected from the group consisting of propylene glycol, phenoxyethanol, methanol, ethanol, isopropyl alcohol, and mixtures thereof. In a preferred embodiment they are selected from the group propylene glycol, phenoxyethanol, and mixtures thereof; still more preferred is a mixture of propylene gylcol and phenoxyethanol.

Surfactants useful in the compositions and uses of the present invention include those selected from the group consisting of sarcosinates, pluronic F68, sodium lauryl sulfate, sorbitan monolaurate, lauryldimethylamineoxide, lauric-diethanolamide, PEG-Esters (polyethylene glycol-dilaurate), coconut hydroxyethyl imidazoline, sodium sulfosuccinate ester of lauric MEA, sodium sulfosuccinate ester of ethoxylated lauryl alcohol, lauric-monoethanolamide, bis-(2-hydroxyethyl) cocoamine oxide·IPA, bis-(2-hydroxyethyl) tallowamine oxide·IPA, dimethylcocoamine oxide·IPA, dimethylcocoamine oxide, (negative inhibitors), polyoxypropylene bases, coconut fatty acid, 2-sulfo-ester, sodium salt, N-coconut oil acyl-N-methyl taurine, sodium salt, lauroyl sarcosine, 30% sodium lauryl sarcosinate, sodium lauroyl sarcosinate, myristoyl sarcosine, oleoyl sarcosine, stearoyl sarcosine, polyoxyethelene 21 stearyl ether (0.1% BHA & 0.005% citric acid as preservatives), lauroamphoglycinate, lauroamphocarboxyglycinate, lauroamphocarboxypropinate, lauroamphocarboxyglycinate-sulfanate, sodium lauryl sulfate (66% lauryl, 27% myristyl, 71% cetyl), polyoxyethylene sorbitan mono-oleate, and mixtures thereof. In a preferred embodiment they are sarcosinates, amine oxides, pluronic F68, sodium lauryl sulfate, and mixtures thereof, more preferred are pluronic F68, sodium lauryl sulfate, amine oxides, and mixtures theeof.

Chelating agents useful in the compositions and uses of the present invention include those selected from the group consisting of EDTA, EDTA (Na)₂, EDTA (Na)₄, TEA, lactic acid, citric acid and mixtures thereof. In a preferred embodiment, they are lactic acid, citric acid, EDTA(Na)₂, EDTA (Na)₄, TEA, and mixtures thereof, more preferred are lactic acid, EDTA(Na)₂, EDTA(Na)₄, citric acid and mixtures thereof, highly preferred are lactic acid, EDTA(Na)₂, EDTA(Na)₄, and mixtures thereof.

Parabens useful in the compositions and uses of the present invention may be selected from the group consisting of methyl and propyl parabens. These may possess the methyl alone or in combination with 1 propyl paraben; 4 methyl 1 propyl; and mixtures thereof (and ester).

Water q.s. may form the remainder of the carrier and is selected from the group consisting of sterile water, distilled water, deionized water, tap and well water. In a preferred embodiment, they are sterile water, distilled water or deionized water. Emulsions may also be employed.

The alcohols discussed above may be employed in the compositions and uses of the present invention at any suitable level. In a preferred embodiment, they are present at a level of 5 to 60%, more preferred at 10 to 30% and, in a highly preferred embodiment, at 20 to 25%, by weight per volume of solution.

The surfactant discussed above may be employed in the compositions and uses of the present invention at any suitable level. In a preferred embodiment, they are present at a level of 0.25 to 10%, more preferred at 2 to 10% and, highly preferred, at 4 to 8%, weight per volume of solution.

The parabens discussed above may be employed in the compositions and uses of the present invention at any suitable level. In a preferred embodiment, they are present at a level of 0.05 to 0.5%, more preferred, at 0.1 to 0.5% and, highly preferred, at 0.1 to 0.3%, weight per volume of solution.

The chelators discussed above may be employed in the compositions of the present invention at any suitable level. In a preferred embodiment, they are present at a level of 0.05 to 3%, more preferred at 0.1 to 0.5% and, highly preferred, at 0.1 to 0.2%, weight per volume of solution.

The compositions of the present invention may additionally employ adjunct components conventionally found in pharmaceutical compositions, in their art-established fashion and at their art-established levels. Thus, for example, the compositions may contain additional compatible pharmaceutically active material for combination therapy (such as supplementary antimicrobial, antipruritics, astringents, local anaesthetics, or anti-inflammatory agents), or may contain materials useful in physically formulating various dosage forms of the present invention, such as excipients, dyes, perfumes, thickening agents, stabilizers, skin penetration enhancers, preservatives, or antioxidants.

Topical treatment regimens using the compositions of this invention comprise applying a safe and effective amount of the compositions described herein directly to the infected or at-risk skin or mucous membrane; particularly, in a veterinary use, on the teats, eyes, ear areas or at any other situs particularly susceptible to microbial contamination. The solution may be eg. sprayed, dipped, wiped, dropped, poured or towelled onto the area to be treated. Application can be made once, or preferably several times daily, to prevent eg. bacteria, fungi or mold from forming on the animal's skin, teats, ears and eyes.

The following examples illustrate the invention.

### EXAMPLE 1

The following formulae have been found to be active against a substantial group of organisms. Also, these formulae have been found to be substantially non-irritating as an ear/eye preparation.

| | |
|---|---|
| Ethoxylated Glycerol Monolaurin (Monolaurin EO) | 0.05 - 1.0% |
| Caprylic Acid/Capric Acid Mixture | 0.1 - 5.0% |
| Parabens | 0.0 - 0.3% |
| Pluronic F68 | 1.0 - 10% |
| Phenoxylethanol | 0.0 - 1.0% |
| EDTA (Na)₂ | 0.05 - 0.3% |
| Water | 82.4 - 98.65% |
| Glycerol monolaurin is ethoxylated by about one mole ethylene oxide per mole of glycerol monolaurin by conventional ethoxylation methods as described herein. | |

### EXAMPLE 2

The following concentrate formula has been found to be active against a selective group of organisms, especially bovine mastitis. Also, the concentrate formula has been found to be non-irritating when applied as a teat dip.

| | |
|---|---|
| Ethoxylated Glycerol Monolaurin | 0.5 - 2.0% |
| Caprylic and Capric Acid Mixture | 0.5 - 8.0% |
| Phenoxyethanol | 0.0 - 2.5% |
| Propylene Glycol | 10 -30% |
| Parabens | 0-.0 - 1.0% |
| Pluronic F68 | 5 - 13.0% |
| EDTA (Na)₂/Lactic Acid | 1 - 15% |
| H₂O | 30 - 80% |
| Monolaurin is ethoxylated by about 0.5 to about 1.5 mole of ethylene oxide by conventional methods. | |

Also, the above formulas (Examples I and II) may be used in human or veterinary protocols, e.g., in the treatment of nasal tissue disease, ophthalmic disease, fungal corneal infection of horses, pulmonary disease, genital infections, and otitis externa. The formulas may be used in insectidical or germicidal formulation on human or animal skin and plants, catheters, in egg washing, diapers, and wood preservatives. The formulas are also effective in combating fowl mites, ear mites, and ticks.

The above formulas are effective against yeasts, gram negative and gram positive organisms and protozoan, more particularly: C. albicans, C. parapsilosis, S. cerevisiae, E. coli, Ps. aeruginosu, S. epidermis, S. aureus, Bacillus subtilis, Streptococcus faecalis, Streptacoccus pyogenec, Corynebacterium, Strep mutans, and Trichomonus vaginalis.

### EXAMPLE 3

The following formula concentrate was tested against a yeast, a gram negative and a gram positive organism:

| | % Weight |
|---|---|
| (Monolaurin EO) Ethoxylated Glycerol Monolaurin | 1.0 |
| Caprylic and Capric Mixture | 1.5 |
| Propylene Glycol | 22 |
| Parabens | 0.5 |
| Phenoxyethanol | 2.5 |
| Pluronic F-68 | 5.0 |
| EDTA (NA)₂ | 2.0 |
| dH₂O | q.s. |

After the formula was diluted 1:10 in water, the dilute was introduced to E. coli and to C. parapsilosis organisms. The results are as follows:

| E. COLI | |
|---|---|
| Time | Colony Forming Units/ml |
| Tₒ | 4.3x10⁶ |
| T=2 mins | <30 |
| T=10 mins | <30 |

| C. PARAPSILOSIS | |
|---|---|
| Time | Colony Forming Units/ml |
| Tₒ | 1.7x10⁶ |
| T=2 min. | 2.5x10³ |
| T=10 min. | <30 |

The formula of Example III was diluted 1:20 in water and then the dilute was introduced to S. aureus. The results are as follows:

| S. AUREUS | |
|---|---|
| Time | Colony Forming Units/ml |
| Tₒ | 1.6x10⁶ |
| T=2 min. | <5.0x10¹ |
| T=10 min. | <30 |

The above formula of Example III was diluted 1:8 with water and prophylactically applied to a cow's teats twice a day. As a result, upon analyzing the cow's teats, no substantial growth of micro-organisms was found on the teats. The same treatment was conducted on a cow's teats when the ambient air temperature was substantially below 40°F, and substantially similar results were found. It will be appreciated that substantial growth would normally be found.

### EXAMPLE 4

| Formula 4-1 | |
|---|---|
| | % Weight |
| Monolaurin | 1.0 |
| (Monolaurin EO) Ethoxylated Glycerol Monolaurin | 1.75 |
| Caprylic and Capric Mixture | 5.0 |
| Pluronic F-68 | 13.0 |
| EDTA (NA)₂ | 4.0 |
| H₂O | 75.25 |

| Formula 4-2 | |
|---|---|
| | % Weight |
| (Monolaurin EO) Ethoxylated Glycerol Monolaurin | 1.75 |
| Caprylic and Capric Mixture | 6.0 |
| Pluronic F-68 | 13.0 |
| Lactic Acid (85%) | 15.0 |
| H₂O | 64.25 |

After the formulas of Example 4-1 and 4-2 were diluted 1:8 in water the dilute was introduced to Strep Agalactial and Staph Aureus organisms. The results illustrate the log reduction of organisms after two minutes of exposure with the dilute. Also listed are the log reduction results of other solutions used in the field.

| Log Reduction of Organisms | | | |
|---|---|---|---|
| | Time | Strep Agalactial | Staph Aureus |
| Example 4-1 | T=2 min. | 3.1 | 2.7 |
| Example 4-2 | T=2 min. | 4.3 | 5.1 |
| Tegragon (Quaternary complex) | T=2 min. | 1.0 | 2.0 |
| Teat Care (Chlorhexadine) | T=2 min. | 1.7 | 2.0 |
| All Day (Sorbic Acid) | T=2 min. | - | 1.8 |
| Quartermate (2000 Lodophor) | T=2 min. | 2.2 | 2.4 |

### EXAMPLE 5

The following formula was tested against the identified organisms with the following results.

| Formula 5 | |
|---|---|
| | % Weight |
| (Monolaurin EO) Ethoxylated Glycerol Monolaurin | 0.15 |
| Caprylic and Capric Mixture | 0.30 |
| Parabens | 0.05 |
| Dowanol | 0.1 |
| Pluronic F-68 | 5.0 |
| EDTA (Na)₂ | 0.1 |
| EDTA (Na)₄ | 0.1 |
| d H₂O | 94.2 |

| Ps AERUGINOSA | |
|---|---|
| Time | Colony Forming Units/ml |
| Tₒ | 1.2x10⁶ |
| T=2 min. | <30 |
| T=10 min. | <30 |

| E. COLI | |
|---|---|
| Time | Colony Forming Units/ml |
| Tₒ | 3.0x10⁶ |
| T=2 min. | <30 |
| T=10 min. | <30 |

### EXAMPLE 6

The following formula was tested against the identified organism with the following results.

| | % Weight |
|---|---|
| (Monolaurin EO) Ethoxylated Glycerol Monolaurin | 0.15 |
| Caprylic and Capric Mixture | 0.30 |
| Dowanol | 0.10 |
| Pluronic F-68 | 5.0 |
| EDTA (Na)₂ | 0.1 |
| EDTA (Na)₄ | 0.1 |
| d H₂O | 94.25 |

| Ps AERUGINOSA | |
|---|---|
| Time | Colony Forming Units/ml |
| Tₒ | 1.2x10⁶ |
| T₂ | <30 |
| T₁₀ | <30 |

| E. COLI | |
|---|---|
| Time | Colony Forming Units/ml |
| Tₒ | 3.0x10⁶ |
| T=2 min. | < 30 |
| T=10 min. | < 30 |

## Claims (Claims for the following Contracting State(s): BE, CH, DE, FR, GB, IT, LI, NL)

1. A topical antimicrobial pharmaceutical composition comprising a tertiary mixture comprising:
(a) a glyceryl fatty acid ester;
(b) a mixture of fatty acids comprising:
(i) a first fatty acid antimicrobial agent which is a C₆-C₁₈ fatty acid; and
(ii) a second fatty acid antimicrobial agent which is a C₆-C₁₈ fatty acid; and
(c) a pharmaceutically acceptable carrier;
wherein the ratio by weight of the ester to the first and second fatty acids combined is 1:10 to 1:5.

2. A topical antimicrobial pharmaceutical composition according to claim 1 wherein the first fatty acid and/or the second fatty acid is a straight chain fatty acid having from six to twelve carbon atoms.

3. A topical antimicrobial pharmaceutical composition according to claim 1 or 2 wherein the glyceryl fatty acid ester is a monoester.

4. A topical antimicrobial pharmaceutical composition according to claim 3 wherein the monoester contains 6 to 21 carbon atoms.

5. A topical antimicrobial pharmaceutical composition according to claim 4 wherein the monoester is monocaprylin, monocaprin, monolaurin, monomyristin, monopalmitolein, monolein, monoicosenoin, monoerucin or a mixture thereof.

6. A topical antimicrobial pharmaceutical composition according to any one of claims 3 to 5 wherein the monoester is present in an amount of 0.025 to 20.0% by weight of the composition.

7. A topical antimicrobial pharmaceutical composition according to any one of claims 1 to 6 in which the carrier comprises an alcohol, chelating agent, surfactant, paraben, water, or a mixture thereof.

8. A topical antimicrobial pharmaceutical composition according to claim 7 wherein the pharmaceutical carrier comprises an alcohol which is propylene glycol, phenoxyethanol, methanol, ethanol, isopropyl alcohol, or a mixture thereof; a chelating agent which is EDTA, Na₂(EDTA), Na₄(EDTA), TEA, lactic acid, citric acid or a mixture thereof; a surfactant which is a sarcosinate, an amine oxide, pluronic 68, sodium lauryl sulfate or a mixture thereof; or a paraben which is methyl paraben, propyl paraben or a mixture thereof.

9. A topical antimicrobial pharmaceutical composition according to claim 7 or 8 which comprises 5% to 60% by volume of the alcohol; 0.25% to 10% by weight of the surfactant; 0.05% to 5% by weight of the parabens; 0.05% to 4% by weight of the chelating agent.

10. A composition according to any one of claims 1 to 9 for use as a topical antimicrobial agent.

11. Use of a composition according to any one of claims 1 to 10 in the preparation of a topical antimicrobial medicament

## Claims (Claims for the following Contracting State(s): ES)

1. A method for preparing a topical antimicrobial pharmaceutical composition comprising a tertiary mixture, said method comprising combining:
(a) a glyceryl fatty acid ester; with
(b) a mixture of fatty acids comprising:
(i) a first fatty acid antimicrobial agent which is a C₆-C₁₈ fatty acid; and
(ii) a second fatty acid antimicrobial agent which is a C₆-C₁₈ fatty acid; and
(c) a pharmaceutically acceptable carrier;
wherein the ratio by weight of the ester to the first and second fatty acids combined is 1:10 to 1:5.

2. A method according to claim 1 wherein the first fatty acid and/or the second fatty acid is a straight chain fatty acid having from 6 to 12 carbon atoms.

3. A method according to claim 1 or 2 wherein the glyceryl fatty acid ester is a monoester.

4. A method according to claim 3 wherein the monoester contains 6 to 21 carbon atoms.

5. A method according to claim 4 wherein the monoester is monocaprylin, monocaprin, monolaurin, monomyristin, monopalmitolein, monolein, monoicosenoin, monoerucin or a mixture thereof.

6. A method according to any one of claims 3 to 5 wherein the monoester is present in an amount of 0.025 to 20.0% by weight of the composition.

7. A method according to any one of claims 1 to 6 in which the carrier comprises an alcohol, chelating agent, surfactant, paraben, water, or a mixture thereof.

8. A method according to claim 7 wherein the pharmaceutical carrier comprises an alcohol which is propylene glycol, phenoxyethanol, methanol, ethanol, isopropyl alcohol, or a mixture thereof; a chelating agent which is EDTA, Na₂(EDTA), Na₄(EDTA), TEA, lactic acid, citric acid or a mixture thereof; a surfactant which is a sarcosinate, an amine oxide, pluronic 68, sodiul lauryl sulfate or a mixture thereof; or a paraben which is methyl paraben, propyl paraben or a mixture thereof.

9. A method according to claim 7 or 8 which comprises 5% to 60% by volume of the alcohol; 0.25% to 10% by weight of the surfactant; 0.05% to 5% by weight of the parabens; 0.05% to 4% by weight of the chelating agent.

10. A topical antimicrobial pharmaceutical composition comprising a tertiary mixture comprising:
(a) a glyceryl fatty acid ester;
(b) a mixture of fatty acids comprising:
(i) a first fatty acid antimicrobial agent which is a C₆-C₁₈ fatty acid; and
(ii) a second fatty acid antimicrobial agent which is a C₆-C₁₈ fatty acid; and
(c) a pharmaceutically acceptable carrier;
wherein the ratio by weight of the ester to the first and second fatty acids combined is 1:10 to 1:5.

11. A topical antimicrobial pharmaceutical composition according to claim 10 wherein the first fatty acid and/or the second fatty acid is a straight chain fatty acid having from 6 to 12 carbon atoms.

12. A topical antimicrobial pharmaceutical composition according to claim 10 to 11 wherein the glyceryl fatty acid ester is a monoester.

13. A topical antimicrobial pharmaceutical composition according to claim 12 wherein the monoester contains 6 to 21 carbon atoms.

14. A topical antimicrobial pharmaceutical composition according to claim 13 wherein the monoester is monocaprylin, monocaprin, monolaurin, monomyristin, monopalmitolein, monolein, monoicosenoin, monoerucin or a mixture thereof.

15. A topical antimicrobial pharmaceutical composition according to any one of claims 12 to 14 wherein the monoester is present in an amount of 0.025 to 20.0% by weight of the composition.

16. A topical antimicrobial pharmaceutical composition according to any one of claims 10 to 15 in which the carrier comprises an alcohol, chelating agent, surfactant, paraben, water, or a mixture thereof.

17. A topical antimicrobial pharmaceutical composition according to claim 16 wherein the pharmaceutical carrier comprises an alcohol which is propylene glycol, phenoxyethanol, methanol, ethanol, isopropyl alcohol, or a mixture thereof; a chelating agent which is EDTA, Na₂(EDTA), Na₄(EDTA), TEA, lactic acid, citric acid or a mixture thereof; a surfactant which is a sarcosinate, an amine oxide, pluronic 68, sodium lauryl sulfate or a mixture thereof; or a paraben which is methyl paraben, propyl paraben or a mixture thereof.

18. A topical antimicrobial pharmaceutical composition according to claim 16 or 17 which comprises 5% to 60% by volume of the alcohol; 0.25% to 10% by weight of the surfactant; 0.05% to 5% by weight of the parabens; 0.05% to 4% by weight of the chelating agent.

19. A composition according to any one of claims 10 to 18 for use as a topical antimicrobial agent.

20. Use of a composition according to any one of claims 10 to 19 in the preparation of a topical antimicrobial medicament.

## Patentansprüche (Patentansprüche für folgende(n) Vertragsstaat(en): BE, CH, DE, FR, GB, IT, LI, NL)

1. Eine topische, antimikrobielle pharmazeutische Zusammensetzung enthaltend ein tertiäres Gemisch, welches umfaßt:
a) einen Glycerylfettsäureester;
b) ein Gemisch aus Fettsäuren umfassend:
(i) ein erstes Fettsäure antimikrobielles Agens, welches eine C₆- bis C₁₈-Fettsäure ist; und
ii) ein zweites Fettsäure antimikrobielles Agens, welches eine C₆-bis C₁₈-Fettsäure ist; und
c) einen pharmazeutisch verträglichen Trägerstoff;
wobei das auf das Gewicht bezogene Verhältnis des Esters zu der Kombination aus der ersten und der zweiten Fettsäure 1 : 10 bis 1 : 5 beträgt.

2. Eine topische, antimikrobielle pharmazeutische Zusammensetzung nach Anspruch 1, in der die erste Fettsäure und/oder die zweite Fettsäure eine geradkettige Fettsäure mit 6 bis 12 Kohlenstoffatomen ist.

3. Eine topische, antimikrobielle pharmazeutische Zusammensetzung nach Anspruch 1 oder 2, in der der Glycerylfettsäureester ein Monoester ist.

4. Eine topische, antimikrobielle pharmazeutische Zusammensetzung nach Anspruch 3, in der der Monoester 6 bis 21 Kohlenstoffatome enthält.

5. Eine topische, antimikrobielle pharmazeutische Zusammensetzung nach Anspruch 4, in der der Monoester Monocaprylin, Monocaprin, Monolaurin, Monomyristin, Monopalmitolein, Monolein, Monoicosenoin, Monoerucin oder ein Gemisch derselben ist.

6. Eine topische, antimikrobielle pharmazeutische Zusammensetzung nach einem der Ansprüche 3 bis 5, in der der Monoester in einer Menge von 0,025 bis 20 Gew.-% der Zusammensetzung vorhanden ist.

7. Eine topische, antimikrobielle pharmazeutische Zusammensetzung nach einem der Ansprüche 1 bis 6, in der der Trägerstoff einen Alkohol, Chelatbildner, Surfactant, Paraben, Wasser oder ein Gemisch derselben umfaßt.

8. Eine topische, antimikrobielle pharmazeutische Zusammensetzung nach Anspruch 7, in der der pharmazeutische Trägerstoff einen Alkohol, welcher Propylenglykol, Phenoxyethanol, Methanol, Ethanol, Isopropylalkohol, oder ein Gemisch derselben ist; einen Chelatbildner, welcher EDTA, Na₂(EDTA), Na₄(EDTA), TEA, Milchsäure, Zitronensäure oder ein Gemisch derselben ist; ein Surfactant, welches ein Sarcosinat, ein Aminoxid, Pluronic 68, Natriumlaurylsulfat oder ein Gemisch derselben ist; oder ein Paraben, welches Methylparaben, Propylparaben oder ein Gemisch derselben ist, umfaßt.

9. Eine topische, antimikrobielle pharmazeutische Zusammensetzung nach den Ansprüchen 7 oder 8, welche 5 bis 60 Vol.% des Alkohols; 0,25 bis 10 Gew.-% des Surfactants; 0,05 bis 5 Gew.-% des Parabens; 0,05 bis 4 Gew.-% des Chelatbildners umfaßt.

10. Eine Zusammensetzung nach einem der Ansprüche 1 bis 9 zur Verwendung als ein topisches, antimikrobielles Agens.

11. Verwendung einer Zusammensetzung nach jeglichem der Ansprüche 1 bis 10 für die Herstellung eines topischen, antimikrobiellen Medikamentes.

## Patentansprüche (Patentansprüche für folgende(n) Vertragsstaat(en): ES)

1. Verfahren zur Herstellung einer topischen, antimikrobiellen pharmazeutischen Zusammensetzung enthaltend ein tertiäres Gemisch, bei dem man:
a) einen Glycerylfettsäureester; mit
b) einem Gemisch aus Fettsäuren umfassend:
(i) ein erstes Fettsäure antimikrobielles Agens, welches eine C₆- bis C₁₈-Fettsäure ist; und
ii) ein zweites Fettsäure antimikrobielles Agens, welches eine C₆-bis C₁₈-Fettsäure ist; und
c) einem pharmazeutisch verträglichen Trägerstoff kombiniert;
wobei das auf das Gewicht bezogene Verhältnis des Esters zu der Kombination aus der ersten und der zweiten Fettsäure 1 : 10 bis 1 : 5 beträgt.

2. Verfahren nach Anspruch 1, bei dem die erste Fettsäure und/oder die zweite Fettsäure eine geradkettige Fettsäure mit 6 bis 12 Kohlenstoffatomen ist.

3. Verfahren nach Anspruch 1 oder 2, bei dem der Glycerylfettsäureester ein Monoester ist.

4. Verfahren nach Anspruch 3, bei dem der Monoester 6 bis 21 Kohlenstoffatome enthält.

5. Verfahren nach Anspruch 4, bei dem der Monoester Monocaprylin, Monocaprin, Monolaurin, Monomyristin, Monopalmitolein, Monolein, Monoicosenoin, Monoerucin oder ein Gemisch derselben ist.

6. Verfahren nach einem der Ansprüche 3 bis 5, bei dem der Monoester in einer Menge von 0,025 bis 20 Gew.-% der Zusammensetzung vorhanden ist.

7. Verfahren nach einem der Ansprüche 1 bis 6, bei dem der Trägerstoff einen Alkohol, Chelatbildner, Surfactant, Paraben, Wasser oder ein Gemisch davon umfaßt.

8. Verfahren nach Anspruch 7, bei dem der pharmazeutische Trägerstoff einen Alkohol, welcher Propylenglykol, Phenoxyethanol, Methanol, Ethanol, Isopropylalkohol, oder ein Gemisch derselben ist; einen Chelatbildner, welcher EDTA, Na₂(EDTA), Na₄(EDTA), TEA, Milchsäure, Zitronensäure oder ein Gemisch derselben ist; ein Surfactant, welches ein Sarcosinat, ein Aminoxid, Pluronic 68, Natriumlaurylsulfat oder ein Gemisch derselben ist; oder ein Paraben, welches Methylparaben, Propylparaben oder ein Gemisch derselben ist, umfaßt.

9. Verfahren nach den Ansprüchen 7 oder 8, welches 5 bis 60 Vol.% des Alkohols; 0,25 bis 10 Gew.-% des Surfactants; 0,05 bis 5 Gew.-% des Parabens; 0,05 bis 4 Gew.-% des Chelatbildners umfaßt.

10. Eine topische, antimikrobielle pharmazeutische Zusammensetzung enthaltend ein tertiäres Gemisch, welches umfaßt:
a) einen Glycerylfettsäureester;
b) ein Gemisch aus Fettsäuren umfassend:
(i) ein erstes Fettsäure antimikrobielles Agens, welches eine C₆- bis C₁₈-Fettsäure ist; und
ii) ein zweites Fettsäure antimikrobielles Agens, welches eine C₆-bis C₁₈-Fettsäure ist; und
c) einen pharmazeutisch verträglichen Trägerstoff;
wobei das auf das Gewicht bezogene Verhältnis des Esters zu der Kombination aus der ersten und der zweiten Fettsäure 1 : 10 bis 1 : 5 beträgt.

11. Eine topische, antimikrobielle pharmazeutische Zusammensetzung nach Anspruch 10, in der die erste Fettsäure und/oder die zweite Fettsäure eine geradkettige Fettsäure mit 6 bis 12 Kohlenstoffatomen ist.

12. Eine topische, antimikrobielle pharmazeutische Zusammensetzung nach Anspruch 10 oder 11, in der der Glycerylfettsäureester ein Monoester ist.

13. Eine topische, antimikrobielle pharmazeutische Zusammensetzung nach Anspruch 12, in der der Monoester 6 bis 21 Kohlenstoffatome enthält.

14. Eine topische, antimikrobielle pharmazeutische Zusammensetzung nach Anspruch 13, in der der Monoester Monocaprylin, Monocaprin, Monolaurin, Monomyristin, Monopalmitolein, Monolein, Monoicosenoin, Monoerucin oder ein Gemisch derselben ist.

15. Eine topische, antimikrobielle pharmazeutische Zusammensetzung nach einem der Ansprüche 12 bis 14, in der der Monoester in einer Menge von 0,025 bis 20 Gew.-% der Zusammensetzung vorhanden ist.

16. Eine topische, antimikrobielle pharmazeutische Zusammensetzung nach einem der Ansprüche 10 bis 15, in der Trägerstoff einen Alkohol, Chelatbildner, Surfactant, Paraben, Wasser oder ein Gemisch derselben umfaßt.

17. Eine topische, antimikrobielle pharmazeutische Zusammensetzung nach Anspruch 16, in der der pharmazeutische Trägerstoff einen Alkohol, welcher Propylenglykol, Phenoxyethanol, Methanol, Ethanol, Isopropylalkohol, oder ein Gemisch derselben ist; einen Chelatbildner, welcher EDTA, Na₂(EDTA), Na₄(EDTA), TEA, Milchsäure, Zitronensäure oder ein Gemisch derselben ist; ein Surfactant, welches ein Sarcosinat, ein Aminoxid, Pluronic 68, Natriumlaurylsulfat oder ein Gemisch derselben ist; oder ein Paraben, welches Methylparaben, Propylparaben oder ein Gemisch derselben ist, umfaßt.

18. Eine topische, antimikrobielle pharmazeutische Zusammensetzung nach den Ansprüchen 16 oder 17, welche 5 bis 60 Vol.% des Alkohols; 0,25 bis 10 Gew.-% des Surfactants; 0,05 bis 5 Gew.-% des Parabens; 0,05 bis 4 Gew.-% des Chelatbildners umfaßt.

19. Eine Zusammensetzung nach einem der Ansprüche 10 bis 18 zur Verwendung als ein topisches, antimikrobielles Agens.

20. Verwendung einer Zusammensetzung nach jeglichem der Ansprüche 10 bis 19 für die Herstellung eines topischen, antimikrobiellen Medikamentes.

## Revendications (Revendications pour l'(les) Etat(s) contractant(s) suivant(s): BE, CH, DE, FR, GB, IT, LI, NL)

1. Composition pharmaceutique antimicrobienne topique comprenant un mélange ternaire comprenant
(a) un ester d'acide gras du glycérol;
(b) un mélange d'acides gras comprenant :
(i) un premier agent antimicrobien de type acide gras qui est un acide gras en C₆₋₁₈, et
(ii) un deuxième agent antimicrobien de type acide gras qui est un acide gras en C₆₋₁₈, et
(c) un support pharmaceutiquement acceptable ;
le rapport en poids de l'ester aux premier acide gras et second acide gras combinés étant de 1/10 à 1/5.

2. Composition pharmaceutique antimicrobienne topique selon la revendication 1, dans laquelle le premier acide gras et/ou le second acide gras est un acide gras à chaîne linéaire comportant de 6 à 12 atomes de carbone.

3. Composition pharmaceutique antimicrobienne topique selon la revendication 1 ou 2 dans laquelle l'ester d'acide gras du glycérol est un monoester.

4. Composition pharmaceutique antimicrobienne topique selon la revendication 3 dans laquelle le monoester contient de 6 à 21 atomes de carbone.

5. Composition pharmaceutique antimicrobienne topique selon la revendication 4 dans laquelle le monoester est la monocapryline, la monocaprine, la monolaurine, la monomyristine, la monopalmitoléine, la monoléine, la monoicosénoïne, la monoérucine ou un mélange de ces composés.

6. Composition pharmaceutique antimicrobienne topique selon l'une quelconque des revendications 3 à 5 dans laquelle le monoester est présent à raison de 0,025 à 20,0 % en poids de la composition.

7. Composition pharmaceutique antimicrobienne topique selon l'une quelconque des revendications 1 à 6 dans laquelle le support comprend un alcool, un agent chélateur, un agent tensio-actif, un parabène, de l'eau ou un mélange de ces composés.

8. Composition pharmaceutique antimicrobienne topique selon la revendication 7 dans laquelle le support pharmaceutique comprend un alcool qui est le propylèneglycol, le phénoxyéthanol, le méthanol, l'éthanol, l'alcool isopropylique ou un mélange de ces composés, un agent chélateur qui est l'EDTA, le Na₂(EDTA), le Na₄(EDTA), le TEA, l'acide lactique, l'acide citrique ou un mélange de ces composés, un agent tensioactif qui est un sarcosinate, un oxyde d'amine, le Pluronic 68, le laurylsulfate de sodium ou un mélange de ces composés, ou un parabène qui est le méthylparabène, le propylparabène ou un mélange de ces composés.

9. Composition pharmaceutique antimicrobienne topique selon la revendication 7 ou 8 qui comprend 5 % à 60 % en volume de l'alcool, 0,25 % à 10 % en poids de l'agent tensioactif, 0,05 à 5 % en poids du parabène et 0,05 % à 4 % en poids de l'agent chélateur.

10. Composition selon l'une quelconque des revendications 1 à 9 destinée à être utilisée comme agent antimicrobien topique.

11. Utilisation de la composition selon l'une quelconque des revendications 1 à 10 pour la préparation d'un médicament antimicrobien topique.

## Revendications (Revendications pour l'(les) Etat(s) contractant(s) suivant(s): ES)

1. Procédé de préparation d'une composition pharmaceutique antimicrobienne topique comprenant un mélange ternaire, ledit procédé comprenant le fait de mélanger :
(a) un ester d'acide gras du glycérol avec
(b) un mélange d'acides gras comprenant :
(i) un premier agent microbien de type acide gras qui est un acide gras en C₆₋₁₈,
(ii) un deuxième agent antimicrobien de type acide gras qui est un acide gras en C₆₋₁₈, et avec
(c) un support pharmaceutiquement acceptable;
le rapport en poids de l'ester aux premier acide gras et second acide gras combinés étant de 1/10 à 1/5.

2. Procédé selon la revendication 1 dans lequel le premier acide gras et/ou le deuxième acide gras est un acide gras à chaîne linéaire comportant de 6 à 12 atomes de carbone.

3. Procédé selon la revendication 1 ou 2 dans lequel l'ester d'acide gras du glycérol est un monoester.

4. Procédé selon la revendication 3 dans lequel le monoester contient de 6 à 21 atomes de carbone.

5. Procédé selon la revendication 4 dans lequel le monoester est la monocapryline, la monocaprine, la monolaurine, la monomyristine, la monopalmitoléine, la monoléine, la monoicosénoine, la monoérucine ou un mélange de ces composés.

6. Procédé selon l'une quelconque des revendications 3 à 5 dans lequel le monoester est présent à raison de 0,025 à 20,0 % en poids de la composition.

7. Procédé selon l'une quelconque des revendications 1 à 6 dans lequel le support comprend un alcool, un agent chélateur, un agent tensioactif, un parabène, de l'eau ou un mélange de ces composés.

8. Procédé selon la revendication 7 dans lequel le support pharmaceutique comprend un alcool qui est le propylèneglycol, le phénoxyéthanol, le méthanol, l'éthanol, l'alcool isopropylique ou un mélange de ces composés, un agent chélateur qui est l'EDTA, le Na₂(EDTA), le Na₄(EDTA), le TEA, l'acide lactique, l'acide citrique ou un mélange de ces composés, un agent tensioactif qui est un sarcosinate, un oxyde d'amine, le Pluronic 68, le laurylsulfate de sodium ou un mélange de ces composés, ou un parabène qui est le méthylparabène, le propylparabène ou un mélange de ces composés.

9. Procédé selon la revendication 7 ou 8 qui comprend 5 % à 60 % en volume de l'alcool, 0,25 % à 10 % en poids de l'agent tensioactif, 0,05 à 5 % en poids du parabène et 0,05 % à 4 % en poids de l'agent chélateur.

10. Composition pharmaceutique antimicrobienne topique comprenant un mélange ternaire comprenant:
(a) un ester d'acide gras du glycérol,
(b) un mélange d'acides gras comprenant :
(i) un premier agent anti microbien de type acide gras qui est un acide gras en C₆₋₁₈,
(ii) un deuxième agent antimicrobien de type acide gras qui est un acide gras en C₆₋₁₈, et
(c) un support pharmaceutiquement acceptable;
le rapport en poids de l'ester aux premier acide gras et second acide gras combinés étant de 1/10 à 1/5.

11. Composition pharmaceutique antimicrobienne topique, selon la revendication 10, dans laquelle le premier acide gras et/ou le second acide gras est un acide gras à chaîne linéaire comportant de 6 à 12 atomes de carbone.

12. Composition pharmaceutique antimicrobienne topique selon la revendication 10 ou 11 dans laquelle l'ester d'acide gras du glycérol est un monoester.

13. Composition pharmaceutique antimicrobienne topique selon la revendication 12 dans laquelle le monoester contient de 6 à 21 atomes de carbone.

14. Composition pharmaceutique antimicrobienne topique selon la revendication 13 dans laquelle le monoester est la monocapryline, la monocaprine, la monolaurine, la monomyristine, la monopalmitoléine, la monoléine, la monoicosénoïne, la monoérucine ou un mélange de ces composés.

15. Composition pharmaceutique antimicrobienne topique selon l'une quelconque des revendications 12 à 14 dans laquelle le monoester est présent à raison de 0,025 à 20,0 % en poids de la composition.

16. Composition pharmaceutique antimicrobienne topique selon l'une quelconque des revendications 10 à 15 dans laquelle le support comprend un alcool, un agent chélateur, un agent tensio-actif, un parabène, de l'eau ou un mélange de ces composés.

17. Composition pharmaceutique antimicrobienne topique selon la revendication 16 dans laquelle le support pharmaceutique comprend un alcool qui est le propylèneglycol, le phénoxyéthanol, le méthanol, l'éthanol, l'alcool isopropylique ou un mélange de ces composés, un agent chélateur qui est l'EDTA, le Na₂(EDTA), le Na₄(EDTA), le TEA, l'acide lactique, l'acide citrique ou un mélange de ces composés, un agent tensioactif qui est un sarcosinate, un oxyde d'amine, le Pluronic 68, le laurylsulfate de sodium ou un mélange de ces composés, ou un parabène qui est le méthylparabène, le propylparabène ou un mélange de ces composés.

18. Composition pharmaceutique antimicrobienne topique selon la revendication 16 ou 17 qui comprend 5 % à 60 % en volume de l'alcool, 0,25 % à 10 % en poids de l'agent tensioactif, 0,05 à 5 % en poids du parabène et 0,05 % à 4 % en poids de l'agent chélateur.

19. Composition selon l'une quelconque des revendications 10 à 18 destinée à être utilisée comme agent antimicrobien topique.

20. Utilisation de la composition selon l'une quelconque des revendications 10 à 19 pour la préparation d'un médicament antimicrobien topique.
